# EUROPÄISCHE PATENTSCHRIFT

(11) **EP 4 159 269 B1**
(45) Veröffentlichungstag und Bekanntmachung des Hinweises auf die Patenterteilung: **05.11.2025**
(21) Anmeldenummer: 21200416.2
(22) Anmeldetag: 01.10.2021
(51) Int. Cl.: A61N 1/04, A61B 5/256

(54) **VORRICHTUNG MIT WENIGSTENS EINER ELEKTRODENEINHEIT FÜR EINE ELEKTROSTIMULATION ODER EINE DATENERFASSUNG DIAGNOSTISCHER GERÄTE**
DEVICE COMPRISING AT LEAST ONE ELECTRODE UNIT FOR AN ELECTROSTIMULATION OR A DATA ACQUISITION OF DIAGNOSTIC DEVICES
DISPOSITIF DOTÉ D'AU MOINS UNE UNITÉ D'ÉLECTRODE POUR UNE ÉLECTROSTIMULATION OU UNE SAISIE DE DONNÉES DES APPAREILS DIAGNOSTIQUES

(43) Veröffentlichungstag der Anmeldung: 05.04.2023
(73) Patentinhaber: Pierenkemper GmbH, 35630 Ehringshausen (DE)
(72) Erfinder: Roger, Pierenkemper, 35037 Marburg (DE); Peinze, Jonas, 9485 Nendeln (LI)
(74) Vertreter: Knefel, Cordula

(56) Entgegenhaltungen:
- EP-A2- 0 459 239
- WO-A1-2005/079910
- US-A1- 2019 167 192
- US-B1- 6 341 237

## Beschreibung

Die Erfindung betrifft eine Vorrichtung mit wenigstens einer Elektrodeneinheit für eine Elektrostimulation oder eine Datenerfassung diagnostischer Geräte.

Zum Stand der Technik (DE 20 2012 102 393 U1) gehört eine Vorrichtung zur komplexen Elektromyostimulation. Diese zum Stand der Technik gehörende Vorrichtung umfasst einen Anzug, eine Stimulationseinheit, eine Bedieneinheit sowie eine die Bedieneinheit und die Stimulationseinheit zur Signalübertragung verbindende Kommunikationseinheit. Dieser Anzug besteht aus einem Kettengewirk. Bei diesem Anzug ist die Elektrodeneinheit an dem Anzug angeordnet. Die Elektroden bestehen aus metallisierten Polymerfasern. Die Kontaktelektroden können auch aus leitfähigen Polymermassen bestehen. Diese zum Stand der Technik gehörenden Kontaktelektroden weisen den Nachteil auf, dass die Polymermassen leicht brüchig werden, insbesondere bei intensiver Nutzung des Anzuges. Werden die Polymermassen brüchig, sind die Elektroden nicht mehr funktionsfähig und der Anzug ist für die Elektrostimulation nicht mehr zu gebrauchen. Zudem entsprechen die derzeit am Markt erhältlichen Materialien nicht den hohen Zytotoxizitätsanforderungen, die an Produkte dieser Art gestellt werden.

### Weiterhin gehört zum Stand der Technik

(EP 2 815 787 A2) eine Elektrodenanordnung und Vorrichtung zur Behandlung von Schmerzen.

Diese zum Stand der Technik gehörende Elektrodenanordnung weist einen Schichtaufbau auf, der aus einem Tragelement, welches beispielsweise aus Gewirk oder Textil bestehen kann, und einem fest in der Vorrichtung angeordneten Druckkörper besteht.

Die Positionen der Elektroden des Elektrodenfelds können gemäß diesem Stand der Technik so gewählt werden, dass diese der Form des Körpers, an der das Elektrodenfeld in Anlage gelangen soll, entspricht. Es ist möglich, dass die Verteilung der Elektroden des Elektrodenfelds der Form eines zur Ausübung eines Druckes eines fest vorgesehenen Druckkörpers folgt. Beispielsweise können für unterschiedliche Stellen am Körper unterschiedliche, jedoch fest angeordnete Druckkörper vorgesehen sein. Es sind beispielsweise als Pelotten ausgestaltete Druckkörper für ein Knie oder ein Schultergelenk bekannt. Die Elektroden des Elektrodenfelds können flächig über der Fläche des Druckkörpers verteilt sein.

Diese zum Stand der Technik gehörende Vorrichtung kann weiter verbessert werden, indem die Andruckkraft der Elektrodenanordnung an den Körper eines Trägers individuell eingestellt wird.

Darüber hinaus sind die fest in den Anzügen angeordneten Druckkörper von Nachteil, wenn der Anzug gereinigt wird, da die Druckkörper schlecht trocknen.

### Weiterhin gehört zum Stand der Technik

(WO 2009/043196 A1) eine Textilelektrodenvorrichtung, die ebenfalls einen Schichtaufbau aufweist.

Diese zum Stand der Technik gehörenden textilintegrierten Elektroden für eine transkutane Elektrostimulation (Oberflächenelektrostimulation) werden in Manschetten oder Kleidungsstücken integriert für die Anwendung von Elektrotherapien, muskulärem Aufbautraining, elektrischer Schmerztherapie oder funktioneller Elektrostimulation.

Gemäß diesem Stand der Technik sind Druckkörper vorgesehen, die als aufblasbare Druckkörper oder als Aktuatoren ausgebildet sind. Diese Druckkörper sind sehr aufwendig aufgebaut und verteuern die Textilelektrodenvorrichtung erheblich, da eine Vielzahl von Druckkörpern in einer derartigen Vorrichtung angeordnet werden, nämlich an jeder Stelle, an der eine Elektrodenanordnung vorgesehen ist.

Gemäß dem Stand der Technik (US 6,341,237 B1) ist eine gürtelähnliche Vorrichtung für eine Elektromuskelstimulation offenbart. Diese zum Stand der Technik gehörende Vorrichtung weist eine Elektrodeneinheit auf, die aus wenigstens zwei Lagen gebildet ist. Hinter den Elektroden sind Schwämme angeordnet, die ausgetauscht werden können. Die Schwämme dienen zur Speicherung von Feuchtigkeit und werden durch ein Gitter von leitfähigen Bahnen direkt auf die Haut gedrückt. Die Vorrichtung ist als Band oder Gürtel aus einem elastischen Material gebildet, so dass die Elektroden gegen die Haut des Patienten gepresst werden, um die Position der Elektroden einzustellen. Wenn die Schwämme mit Schmutz und Schweiß verunreinigt werden, können Sie ersetzt werden. Diese zum Stand der Technik gehörende Vorrichtung kann weiter verbessert werden, indem die Elektroden optimiert an der Haut des Patienten anliegen.

### Weiterhin gehört zum Stand der Technik

(US 2019/0167192 A1) eine Vorrichtung zum automatischen Befeuchten einer Textilelektrode. Über den Einsatz von feuchtigkeitsspeichernden Materialien und feuchtigkeitsabweisenden Materialien in einer Tasche wird eine korrekte Befeuchtung der Elektrode erreicht. Auch diese zum Stand der Technik gehörende Vorrichtung kann dahingehend verbessert werden, dass die Elektroden individuell und mit einem optimierten Anpressdruck an der Haut anliegen.

Weiterhin gehört zum Stand der Technik (WO 2005/079910 A1), eine elastische Bandage mit voneinander beabstandeten Elektroden. Diese zum Stand der Technik gehörende Bandage weist an der Außenseite eine, dem elastischen Bereich gegenüber wesentlich geringer elastische Tasche auf, die ein elastisches Kissen als Abstützung der Elektroden enthält. Gemäß diesem Stand der Technik ist entweder ein aufblasbarer Schlauch oder ein Kissen vorgesehen, das Druck auf die Elektroden ausübt.

Diese zum Stand der Technik gehörende Vorrichtung weist den Nachteil auf, dass im Fall des Schaumstoffkissens eine Individualisierung des Anpressdruckes nicht möglich ist. Weiterer Nachteil dieses Standes der Technik ist, dass der Anpressdruck nicht für einzelne Elektroden individuell einstellbar ist, da mit dem elastischen Kissen und mit den Luftschläuchen gleichzeitig mehrere Elektroden unterfüttert werden.

Das der Erfindung zugrundeliegende technische Problem besteht darin, eine Vorrichtung für eine Elektrostimulation oder für eine Datenerfassung diagnostischer Geräte mit wenigstens einer Elektrodeneinheit anzugeben, welche eine individuelle Anpassung des Anpressdruckes der Elektroden erlaubt, welche darüber hinaus preiswert ist und welche einfach zu waschen und zu sterilisieren ist.

Dieses technische Problem wird durch eine Vorrichtung mit den Merkmalen gemäß Anspruch 1 gelöst.

Die erfindungsgemäße Vorrichtung mit wenigstens einer Elektrodeneinheit für eine Elektrostimulation oder eine Datenerfassung diagnostischer Geräte, wobei die Vorrichtung aus einem textilen Material gebildet ist, wobei die Vorrichtung aus wenigstens zwei Lagen gebildet ist, wobei in oder an einer ersten Lage die wenigstens eine Elektrodeneinheit angeordnet ist, und eine zweite Lage derart an der ersten Lage angeordnet ist, dass die erste und die zweite Lage wenigstens eine Tasche bilden, wobei in der Tasche wenigstens ein Unterfütterungsteil austauschbar angeordnet ist, zeichnet sich dadurch aus, dass verschiedene austauschbare Unterfütterungsteile jeweils unterschiedliche Dicken oder Härtegrade aufweisen.

Dadurch dass das wenigstens eine Unterfütterungsteil austauschbar in der Tasche angeordnet ist, können Unterfütterungsteile verwendet werden, die den erforderlichen Anpressdruck der Elektrodeneinheit an dem Körper des Trägers der Vorrichtung erzeugen.

So ist es beispielsweise möglich, wenn die Vorrichtung als Anzug ausgebildet ist, im Bereich eines Hohlkreuzes des Trägers wenigstens ein Unterfütterungsteil vorzusehen, derart, dass die Elektrodeneinheit an der Haut des Trägers der Vorrichtung anliegt.

Es besteht jedoch auch die Möglichkeit, in der Tasche beispielsweise zwei Unterfütterungsteile anzuordnen, beispielsweise rechts und links von der Elektrodeneinheit, so dass eine Zugspannung auf die erste Lage ausgeübt wird und damit der Anpressdruck der Elektrodeneinheit an der Haut des Trägers verringert wird.

In diesem Fall kann durch das Unterfütterungsteil ein Anpressdruck verringert werden.

Ist beispielsweise eine Elektrodeneinheit im Bereich eines Nackens vorgesehen, kann ein Unterfütterungsteil derart in der wenigstens einen Tasche angeordnet werden, dass ein punktueller Druck vorliegt, so dass die Elektrodeneinheit punktuell oder mit einer geringen flächigen Auflage an dem Körper anliegt. Werden beispielsweise in den Randbereichen der Elektrodeneinheit Unterfütterungsteile vorgesehen, kann eine Druckentlastung zwischen den Unterfütterungsteilen erzeugt werden.

Dies kann beispielsweise bei einem Krafttraining der Fall sein, bei dem man im Nackenbereich auf den Schultern einen nicht so großen Druck mit der Elektrodeneinheit ausüben möchte, da sich dieser Druck unangenehm bei einem Krafttraining auswirkt.

Bei anderen Trainings ist es beispielsweise gewünscht, auch im Schulterbereich die wenigstens eine Elektrodeneinheit mit einem größeren Druck an der Haut des Trägers anliegen zu lassen, um eine zusätzliche gute Stimulation zu erreichen.

Die erfindungsgemäße Vorrichtung weist neben dem Vorteil, dass der Anpressdruck der wenigstens einen Elektrodeneinheit individuell durch das wenigstens eine Unterfütterungsteil eingestellt werden kann, den weiteren Vorteil auf, dass das wenigstens eine Unterfütterungsteil aus der wenigstens einen Tasche der Vorrichtung entnommen werden kann und dass damit die Vorrichtung einfach gereinigt, beispielsweise gewaschen und sterilisiert werden kann.

Durch die Entnahme der Unterfütterungsteile können diese gesondert gereinigt und sterilisiert werden.

Sie können auch gesondert getrocknet werden, so dass die Vorrichtung schneller wieder einsatzfähig ist. Die Unterfütterungsteile brauchen nämlich in der Regel länger zum Trocknen als die Vorrichtung selbst.

Darüber hinaus ist die erfindungsgemäße Vorrichtung sehr preiswert, da in der Vorrichtung lediglich wenigstens eine Tasche vorgesehen ist, in der unterschiedliche Unterfütterungsteile in unterschiedlicher Anzahl austauschbar angeordnet werden können.

Es sind keine teuren aufblasbaren Luftkissen oder Aktuatoren oder dergleichen vorgesehen.

Es werden lediglich verschiedene dem Bedarf individuell durch die entsprechende Auswahl angepasste Unterfütterungsteile in der wenigstens einen Tasche angeordnet.

Gemäß einer vorteilhaften Ausführungsform der Erfindung ist vorgesehen, dass die wenigstens eine Tasche derart an der Vorrichtung angeordnet ist, dass die wenigstens eine Elektrodeneinheit wenigstens teilweise von der wenigstens einen Tasche überdeckt ausgebildet ist.

Diese Ausführungsform weist den Vorteil auf, dass das wenigstens eine Unterfütterungsteil unmittelbar einen Anpressdruck der Elektrodeneinheit auf die Haut des Trägers ausübt, da die Tasche die Elektrodeneinheit wenigstens teilweise überdeckt und das Unterfütterungsteil derart in der Tasche angeordnet werden kann, dass die Elektrodeneinheit unmittelbar an die Haut des Trägers gedrückt wird.

Eine weitere vorteilhafte Ausführungsform der Erfindung sieht vor, dass das wenigstens eine Unterfütterungsteil aus einem thermoplastischen Polymer aus Schaumstoff, aus Vlies oder aus Silikon gebildet ist oder dass das wenigstens eine Unterfütterungsteil als Luftkissen oder Abstandsgewirk ausgebildet ist.

Bei der Ausbildung als Luftkissen ist dieses vordefiniert ausgebildet und nicht aufblasbar.

Gemäß einer besonders bevorzugten Ausführungsform der Erfindung besteht das Unterfütterungsteil aus einem thermoplastischen Polymer.

Das Unterfütterungsteil kann in diesem Fall als Spritzgussteil besonders preiswert hergestellt werden. Darüber hinaus können die Spritzgussteile in beliebigen Formen hergestellt werden, so dass die Teile vielfältig einsetzbar sind.

Es besteht jedoch genauso die Möglichkeit, das wenigstens eine Unterfütterungsteil aus Schaumstoff, aus Vlies oder aus Silikon zu bilden. Ebenso die Ausbildung als Luftkissen oder Abstandsgewirk ist vorteilhaft, da Unterfütterungsteile aus diesen Materialien einen entsprechenden Druck auf die Elektrodeneinheit ausüben können, wenn sie in der Tasche angeordnet sind oder wie schon beschrieben eine Druckentlastung erzeugen können.

Gemäß einer vorteilhaften Ausführungsform der Erfindung weist die wenigstens eine Tasche eine Fläche auf, die der Fläche der Elektrodeneinheit entspricht.

In diesem Fall ist es möglich, wenigstens ein Unterfütterungsteil derart in der Tasche anzuordnen, dass die Elektrodeneinheit mit einem homogenen Anpressdruck an der Haut des Trägers der Vorrichtung anliegt.

Gemäß einer anderen vorteilhaften Ausführungsform der Erfindung ist vorgesehen, dass die wenigstens eine Tasche eine größere oder eine kleinere Fläche aufweist als die Elektrodeneinheit.

Weist die Tasche eine größere Fläche als die Elektrodeneinheit auf, ist es beispielsweise möglich, am Rand der Elektrodeneinheit oder außerhalb der Elektrodeneinheit wenigstens ein Unterfütterungsteil anzuordnen, so dass eine Druckentlastung erzielt wird.

Weist die Tasche eine kleinere Fläche auf, kann punktuell ein höherer Druck auf die Elektrodeneinheit mit wenigstens einem Unterfütterungsteil erzeugt werden.

Gemäß einer besonders bevorzugten Ausführungsform der Erfindung ist vorgesehen, dass in der wenigstens einen Tasche wenigstens ein Unterfütterungsteil angeordnet ist, derart, dass die wenigstens eine Elektrodeneinheit mit einem vorgegebenen Druck an einem Körper des Trägers anliegend ausgebildet ist.

Durch die Anordnung des wenigstens einen Unterfütterungsteils in der wenigstens einen Tasche kann der Druck, mit dem die Elektrodeneinheit an der Haut des Trägers anliegt, frei gewählt werden. Es können beispielsweise ein oder mehrere Unterfütterungsteile nebeneinander- oder übereinanderliegend, bezogen auf eine Orthogonale der Elektrodeneinheit in der wenigstens einen Tasche angeordnet sein.

Ist der Anpressdruck zu gering, können mehrere Unterfütterungsteile übereinanderliegend in der wenigstens einen Tasche angeordnet werden. Es besteht auch die Möglichkeit, ein Unterfütterungsteil zu verwenden, welches eine größere Dicke aufweist.

Gemäß einer vorteilhaften Ausführungsform der Erfindung können die Unterfütterungsteile einen konstanten Querschnitt aufweisen. Es besteht auch die Möglichkeit, einen nicht konstanten Querschnitt der Unterfütterungsteile vorzusehen.

Gemäß der Erfindung ist vorgesehen, dass verschiedene austauschbare Unterfütterungsteile jeweils unterschiedliche Dicken und/oder Härtegrade aufweisen.

Die Unterfütterungsteile können unterschiedliche Dicken aufweisen, so dass ein Unterfütterungsteil gewählt werden kann, welches den erforderlichen Anpressdruck gewährleistet.

Die Unterfütterungsteile können auch unterschiedliche Härtegrade aufweisen, um hierüber den erforderlichen Anpressdruck einzustellen.

Eine weitere vorteilhafte Ausführungsform der Erfindung sieht vor, dass die zwei Lagen der Vorrichtung zu wenigstens einer Tasche zusammengefügt sind durch Kleben, Nähen und/oder Verschweißen.

Die Lagen werden im Randbereich der Tasche zusammengefügt. Bei einer Tasche bleibt eine Seite vollständig oder wenigstens teilweise geöffnet, um ein Unterfütterungsteil in der Tasche anordnen zu können.

Die Ränder der Tasche werden vorteilhaft durch Verkleben der beiden Lagen, durch Zusammennähen der beiden Lagen und/oder durch Verschweißen der beiden Lagen gebildet. Es besteht auch die Möglichkeit, andere Verbindungsarten vorzusehen.

Eine Tasche zeichnet sich dadurch aus, dass wenigstens eine Öffnung vorgesehen ist. Durch diese Öffnung kann das wenigstens eine Unterfütterungsteil in der Tasche angeordnet werden oder durch diese Öffnung entnommen werden. Eine Tasche kann auch mehrere Öffnungen aufweisen. Hierdurch wird zum Beispiel ein flächiges Anordnen eines Unterfütterungsteiles erleichtert, wenn eine Tasche beispielsweise zwei sich gegenüberliegende Öffnungen aufweist.

Gemäß einer weiteren vorteilhaften Ausführungsform der Erfindung ist vorgesehen, dass wenigstens eine zusätzliche Tasche vorgesehen ist, die außerhalb eines Bereiches mit einer Elektrodeneinheit an der ersten Lage angeordnet ist.

Durch diese Taschen, die nicht im Bereich einer Elektrodeneinheit angeordnet sind, besteht die Möglichkeit, Unterfütterungsteile anzuordnen und hiermit eine Druckentlastung zu erreichen.

Taschen, die nicht im Bereich einer Elektrodeneinheit angeordnet sind, können beispielsweise auch an diametral sich gegenüberliegenden Seiten des Anzuges vorgesehen sein.

So kann zum Beispiel eine Elektrodeneinheit im Bereich der Lendenwirbel angeordnet sein und eine Tasche im Bauchbereich. Wird im Bauchbereich wenigstens ein Unterfütterungsteil angeordnet, wird ein höherer Anpressdruck des Anzuges im Lendenwirbelbereich erzeugt. Diese Art der Ausbildung ist möglich, wenn Teile der Vorrichtung oder die Vorrichtung selbst eine schlauchförmige Form aufweisen, so dass eine Tasche und ein entsprechendes Unterfütterungsteil in der Tasche eine Zugwirkung auf eine diametral oder annähernd diametral zu der Tasche angeordneten Elektrodeneinheit aufweist.

Gemäß einer besonders bevorzugten Ausführungsform der Erfindung ist die Vorrichtung als Kleidungsstück, insbesondere als textiler Anzug oder als Teil eines textilen Anzuges ausgebildet.

Die Vorrichtung ist besonders vorteilhaft als Anzug ausgebildet, der aus einem textilen Material besteht. Der Anzug liegt zumindest teilweise an der Haut des Trägers an.

Die Vorrichtung kann jedoch auch als Teil eines textilen Anzuges ausgebildet sein, beispielsweise als Armling oder Beinling oder Manschette, als Body oder dergleichen.

Bei dem Anpressdruck handelt es sich um den textilen Anpressdruck der Vorrichtung, beispielsweise des textilen Anzuges.

Der textile Stoff der Vorrichtung, beispielsweise des Anzuges kann elastisch oder nichtelastisch oder als Kombination aus elastischem und nichtelastischem Stoff ausgebildet sein. Auch hierdurch lässt sich der Anpressdruck der Elektrodeneinheit durch Anordnen von wenigstens einem Unterfütterungsteil in wenigstens einer Tasche einstellen.

Gemäß einer weiteren vorteilhaften Ausführungsform der Erfindung weist die Elektrodeneinheit folgende Schichten auf:
a) Grundschicht mit wenigstens einer in oder an der Grundschicht angeordneten Elektrode, und
b) Schicht mit einem der folgenden Schichtaufbauten:
   b1)
      - Kunststoffschicht und
      - Schicht aus Gewirk und
      - Kunststoffschicht
      oder
   b2)
      - Schicht aus Gewirk und
      - Kunststoffschicht
      oder
   b3)
      - Kunststoffschicht und
      - Schicht aus Gewirk
und die Schichten a) und b) sind fest miteinander verbunden.

Die erfindungsgemäße Vorrichtung mit wenigstens einer Elektrodeneinheit kann zum einen für eine Elektrostimulation verwendet werden. Die wenigstens eine Elektrode der Elektrodeneinheit wird mit Strom beaufschlagt und kann aus diesem Grunde für eine Elektrostimulation verwendet werden. Es ist jedoch auch möglich, eine Datenerfassung mit der erfindungsgemäßen Vorrichtung mit der wenigstens einer Elektrodeneinheit durchzuführen, beispielsweise eine Datenerfassung mittels EKG (Elektrokardiogramm), EMG (Elektromyographie) oder zum Beispiel zur Messung von Bioimpedanzen usw..

Die erfindungsgemäße Vorrichtung weist folgenden Aufbau mit folgenden Schichten auf:
a) Grundschicht mit wenigstens einer in oder an der Grundschicht angeordneten Elektrode und
b) Schicht mit einem der folgenden Schichtaufbauten:
   b1)
      - Kunststoffschicht und
      - Schicht aus Gewirk und
      - Kunststoffschicht
      oder
   b2)
      - Schicht aus Gewirk und
      - Kunststoffschicht
      oder
   b3)
      - Kunststoffschicht und
      - Schicht aus Gewirk.

Bei der erfindungsgemäßen Elektrodeneinheit sind die Schichten a) und b) fest miteinander verbunden. Das bedeutet, dass die Schicht a) mit der Schicht b1) oder die Schicht a) mit der Schicht b2) oder die Schicht a) mit der Schicht b3) fest verbunden ist. Vorteilhaft sind die Schichten der Schicht b1) oder der Schicht b2) oder der Schicht b3) ebenfalls fest miteinander verbunden.

Durch diesen Aufbau der Elektrodeneinheit ist gewährleistet, dass die Schicht b) eine Schicht aus einem Gewirk aufweist. Hierdurch bleibt dieser Schichtaufbau stabil, auch wenn die Grundschicht mit der weiteren Schicht häufig in ihrer Grundform verändert, beispielsweise gebogen, geknickt, gestreckt, gestaucht oder dergleichen wird.

Die Elektrodeneinheit kann beispielsweise an der erfindungsgemäße Vorrichtung, beispielsweise einem Anzug für eine Elektrostimulation angeordnet sein. Dieser Anzug enthält vorteilhaft wenigstens eine Elektrodeneinheit. Die wenigstens eine Elektrodeneinheit wird je nach Anordnung an dem Anzug mehr oder weniger stark tordiert oder gestreckt oder gestaucht oder in anderer Weise beansprucht. Durch Ausgestaltung der Elektrodeneinheit mit der Schicht aus Gewirk mit wenigstens einer Kunststoffschicht bleibt die Elektrodeneinheit auch über einen langen Benutzungszeitraum stabil und funktionsfähig, auch bei einer starken mechanischen Beanspruchung.

Gemäß einer vorteilhaften Ausführungsform der Erfindung wird die Elektrode in die Grundschicht eingestickt. Das Einsticken hat den Vorteil, dass die Elektrode in der gewünschten Form in der Grundschicht angeordnet werden kann. Diese gestickte Elektrode ist sehr haltbar.

Gemäß einer vorteilhaften Ausführungsform wird die Elektrode mit elektrisch leitfähigen Fäden in die Grundschicht eingestickt. Vorteilhaft werden die elektrisch leitfähigen Fäden lediglich in einer Oberfläche der Grundschicht eingestickt. Sie werden dabei nicht durch die gesamte Grundschicht durchgestickt. Auf der gegenüberliegenden Seite können nicht elektrisch leitfähige Haltefäden angeordnet sein. Grundsätzlich besteht jedoch auch die Möglichkeit, dass die elektrisch leitfähigen Fäden durch die gesamte Grundschicht durchgehend angeordnet sind.

Gemäß weiteren vorteilhaften Ausführungsformen kann die wenigstens eine Elektrode in der Grundschicht auch durch Drucken, Nähen, Kleben und/oder Einlegen und Fixieren in oder an der Grundschicht angeordnet sein.

Die Elektrode wird vorteilhaft lediglich auf einer Seite der Grundschicht angeordnet. Das heißt, dass die Grundschicht auf einer Seite die elektrisch leitfähige Elektrode aufweist. Auf der anderen Seite ist die Grundschicht nicht oder nur in einem geringen Maße elektrisch leitfähig.

Gemäß einer besonders bevorzugten Ausführungsform der Erfindung ist vorgesehen, dass die wenigstens eine Elektrode in oder an der vorgefertigten Grundschicht angeordnet ist. Diese Ausführungsform weist den Vorteil auf, dass die Elektrode auf der vorgefertigten Grundschicht der Elektrodeneinheit in beliebiger Form aufgestickt werden kann. Die Grundschicht an sich ist fertig hergestellt. Anschließend wird die wenigstens eine Elektrode in oder an dieser vorgefertigten Grundschicht angeordnet. Ist zum Beispiel eine Anpassung von Elektroden an bestimmte Körperregionen erforderlich, kann dieses in dem Stickvorgang festgelegt werden.

Es ist nicht von vornherein notwendig, das Design der gesamten Elektrodeneinheit schon bei der Herstellung der Grundschicht festzulegen. Erst bei der Herstellung der Elektrodeneinheit wird die endgültige Form in der Grundschicht festgelegt. Auf diese Art und Weise können Elektrodeneinheiten mit unterschiedlichen Designs preiswert hergestellt werden.

Die wenigstens eine Elektrode wird vorteilhaft aus wenigstens einem elektrisch leitfähigen Faden gebildet. Es können mehrere Elektroden mit einem Faden oder Fadenstrang hergestellt sein. Diese Elektroden sind dann in Reihe geschaltet. Es besteht auch die Möglichkeit, eine oder mehrere Elektroden aus jeweils einem elektrisch leitfähigen Faden herzustellen. In diesem Fall können eine oder mehrere Elektroden parallel geschaltet werden. Die Elektroden können einzeln oder zusammen oder gruppenweise ansteuerbar ausgebildet werden.

Gemäß einer weiteren vorteilhaften Ausführungsform der Erfindung ist vorgesehen, dass die wenigstens eine Kunststoffschicht elektrisch leitfähig ausgebildet ist. Hierdurch ist es möglich, eine Stromübertragung über die Kunststoffschicht vorzunehmen.

Gemäß einer weiteren vorteilhaften Ausführungsform der Erfindung ist vorgesehen, dass die wenigstens eine Kunststoffschicht aus einem thermoplastischen Material und/oder aus thermoplastischen Elastomeren und/oder aus leitfähigen Silikonen gebildet ist.

Diese Materialien sind besonders flexibel und können mit dem Anzug oder einer Manschette oder dergleichen verformt werden. Darüber hinaus bleibt die Flexibilität über einen sehr langen Zeitraum erhalten.

Gemäß einer weiteren vorteilhaften Ausführungsform der Erfindung ist vorgesehen, dass die wenigstens eine Kunststoffschicht elektrisch leitfähige Partikel aufweist. Hierdurch ist die wenigstens eine Kunststoffschicht ebenfalls elektrisch leitfähig. Durch die Kunststoffschicht kommt der Nutzer der erfindungsgemäßen Vorrichtung, beispielsweise der Patient oder ein Sportler, schneller ins Schwitzen. Durch die vom Schweiß gebildete Feuchtigkeit wird die Leitfähigkeit erhöht.

Es können sämtliche in den Schichten b1), b2) oder b3) angeordneten Kunststoffschichten elektrisch leitfähige Partikel aufweisen. Es besteht jedoch auch die Möglichkeit, dass nur ein Teil der in den Schichten b1), b2) oder b3) angeordneten Kunststoffschichten elektrisch leitfähige Partikel aufweisen.

Die elektrisch leitfähigen Partikel sind vorteilhaft homogen oder wenigstens annähernd homogen in der wenigstens einen Kunststoffschicht angeordnet.

Gemäß einer weiteren vorteilhaften Ausführungsform der Elektrodeneinheit ist vorgesehen, dass die elektrisch leitfähigen Partikel aus Silber und/oder Graphit bestehen. Diese Partikel lassen sich einfach in die Kunststoffschicht einbringen und erhöhen die elektrische Leitfähigkeit der wenigstens einen Kunststoffschicht.

Eine weitere vorteilhafte Ausführungsform der Elektrodeneinheit sieht vor, dass die Grundschicht aus einem Vlies besteht. Es besteht jedoch auch die Möglichkeit, andere Materialien für die Grundschicht zu verwenden. Da die Elektrodeneinheit beispielsweise durch ein Heißpressverfahren hergestellt wird, sollte die Grundschicht hitzebeständig sein. Sie sollte sich bei einem Thermopressverfahren nicht zu sehr zusammendrücken.

Eine weitere vorteilhafte Ausführungsform der Elektrodeneinheit sieht vor, dass die wenigstens eine Kunststoffschicht als Polyurethanfolie ausgebildet ist. Polyurethanfolie lässt sich besonders preiswert herstellen. Darüber hinaus können in der Polyurethanfolie die elektrisch leitfähigen Partikel gut und einfach angeordnet werden.

Eine besonders bevorzugte Ausführungsform der Elektrodeneinheit sieht vor, dass die wenigstens eine Schicht aus Gewirk
- Gaze und/oder
- elastische Gaze und/oder
- gitterförmiges Material und/oder
- gitterförmiges elastisches Material
   aufweist und/oder
- aus Gaze und/oder
- aus elastischer Gaze und/oder
- einem gitterförmigen Material und/oder
- einem gitterförmigen elastischen Material
   gebildet ist.

Das bedeutet, dass in den Schichten b1), b2) oder b3) vorteilhaft eine Schicht aus Gaze und/oder aus elastischer Gaze und/oder einem gitterförmigen Material und/oder einem gitterförmigen elastischen Material gebildet ist.

Die Schichten b1), b2) oder b3) können auch Gaze und/oder gitterförmiges Material, vorzugsweise elastische Gaze und/oder gitterförmiges elastisches Material aufweisen.

Die Gaze und/oder das gitterförmige Material sind vorzugsweise elastisch.

Das Gewirk, beispielsweise die Gaze und/oder das gitterförmige Material, vorzugsweise elastische Material, gewährleisten, dass die wenigstens eine Kunststoffschicht nicht brüchig wird oder höheren mechanischen Belastungen standhält. Diese Schicht aus Gewirk bewirkt eine hohe Stabilität der Elektrodeneinheit bei gleichzeitiger Flexibilität der Elektrodeneinheit, so dass sich die Elektrodeneinheit an Körperteile des Patienten oder Sportlers anpassen kann. Das Gewirk, beispielsweise die Gaze und/oder das gitterförmige Material sind vorteilhaft in einer Richtung, besonders vorteilhaft jedoch in zwei Richtungen, beispielsweise in x- und in y-Richtung elastisch ausgebildet, um eine bestmögliche Anpassung der Elektrodeneinheit an ein Körperteil oder an den Körper eines Patienten oder Sportlers zu gewährleisten.

Vorteilhaft ist das Gewirk, beispielsweise die Gaze oder das gitterförmige Material aus Polyester gebildet. Dieses Material ist preiswert, lang haltbar und lässt sich gut als Gewirk, beispielsweise in Form von Gaze oder gitterförmigem Material mit der entsprechenden Elastizität ausbilden.

Vorteilhaft zeichnet sich die Elektrodeneinheit dadurch aus, dass die Schichten als
- durch ein Thermopressverfahren miteinander verschweißte Schichten oder
- durch ein Thermopressverfahren mit einem Haftvermittler fest verbundene Schichten oder
- durch Aufkaschieren fest miteinander verbundene Schichten oder
- durch ein Hochfrequenzschweißen oder ein Ultraschallschweißen fest miteinander verbundene Schichten
ausgebildet sind.

Die verschiedenen Schichten mittels der vorgenannten Verfahren fest miteinander zu verbinden, weist den Vorteil auf, dass das Verfahren preiswert durchführbar ist und die Flexibilität der Elektrodeneinheit trotzdem gewährleistet ist. Darüber hinaus werden feste, unlösbare Verbindungen zwischen den Schichten gebildet.

Gemäß einer weiteren vorteilhaften Ausführungsform der Elektrodeneinheit ist vorgesehen, dass die Elektrodeneinheit mit der Grundschicht an einem Ganzkörperanzug oder Teilkörperanzug angeordnet ist. Der Ganzkörperanzug deckt den Mittelkörper wie auch die Extremitäten ab, während ein Teilkörperanzug beispielsweise in Form eines T-Shirts, eines Pullovers oder einer Art Hose ausgebildet sein kann. Durch diese Ausbildung ist es möglich, beispielsweise für einen Sportler einen Ganzkörperanzug mit der erfindungsgemäßen Vorrichtung zu versehen, so dass während des Sportes beispielsweise eine Elektrostimulation stattfinden kann.

Gemäß einer anderen vorteilhaften Ausführungsform ist vorgesehen, dass die Elektrodeneinheit mit der Grundschicht auf einer Manschette angeordnet ist. Diese Manschette kann beispielsweise an den Extremitäten eines Sportlers oder eines Patienten angeordnet werden, um ebenfalls bei einer sportlichen Aktivität eine Elektrostimulation durchzuführen oder eine Elektrostimulation für therapeutische Zwecke durchzuführen oder Daten beispielsweise in Form eines EKGs oder EMGs zu erfassen.

Gemäß einer weiteren vorteilhaften Ausführungsform ist vorgesehen, dass die Vorrichtung aus
- Polyester und Elasthan oder
- Polyamid 6 oder Elasthan oder
- Polyamid 6.6 und Elasthan oder
- Polyurethan und Elasthan
besteht.

Diese Materialien sind besonders vorteilhaft, da der Ganz- oder Teilkörperanzug oder die Manschette hierdurch flexibel ausgestaltet sind, um Scherbewegungen, Torsionsbewegungen, Streck- und/oder Stauchbewegungen des Ganz- oder Teilkörperanzuges oder der Manschette bei Bewegung des Sportlers oder Patienten mitzumachen, so dass die Elektrodeneinheit mit der wenigstens einen Elektrode ständig in Körperkontakt bleibt.

Gemäß einer weiteren vorteilhaften Ausführungsform weist die Elektrode einen Widerstand bezüglich der elektrischen Leitfähigkeit von weniger als 100 Ohm pro 10 Zentimeter Länge der Elektrode auf. Dies ist vorteilhaft, da die Elektroden hierdurch eine große Leitfähigkeit aufweisen, so dass eine wirksame Elektrostimulation stattfinden kann.

Eine besonders bevorzugte Ausführungsform der erfindungsgemäßen Vorrichtung mit der wenigstens einen Elektrodeneinheit sieht vor, dass eine gestickte Elektrode verwendet wird, die in einem Heißpressverfahren mit den weiteren Schichten verbunden wird.

Wird lediglich eine Kunststofffolie verwendet, die leitende Partikel enthält, entstehen durch die ständige Bewegung der flächigen Elektrode leicht Beschädigungen. Aus diesem Grunde ist es vorteilhaft, ein Gewirk, beispielsweise in Form einer Gaze oder eines gitterförmigen elastischen Materials zusätzlich vorzusehen.

Die Grundschicht mit der Elektrode ist ebenfalls vorteilhaft niederohmig ausgebildet, das bedeutet sie weist vorteilhaft eine hohe Leitfähigkeit und einen sehr geringen Widerstand auf.

Das Gewirk, beispielsweise die Gaze, vorzugsweise elastisch, oder das gitterförmige, vorzugsweise elastische Material besteht vorteilhaft aus Polyester oder anderen Materialien. Die Gaze ist ebenfalls vorteilhaft elastisch ausgebildet.

Die wenigstens eine Kunststoffschicht besteht vorteilhaft aus einer PU-Folie (Polyurethan), die besonders vorteilhaft elektrisch leitfähige Partikel, beispielsweise Graphit und Silber enthält. Die PU-Folie kann auch aus thermoplastischem Material oder aus thermoplastischen Elastomeren wie auch aus leitfähigen Silikonen bestehen.

Trägt der Sportler einen Ganzkörper- oder Teilkörperanzug, kommt er bei der sportlichen Tätigkeit ins Schwitzen. Hierdurch wird die Grundschicht, die in Richtung der Haut angeordnet ist, mit Schweiß durchsetzt. Durch die Erhöhung der Feuchtigkeit wird auch die Leitfähigkeit verbessert.

Die Kunststoffschichten werden vorteilhaft auf das Gewirk, beispielsweise die Gaze und/oder das gitterförmige elastische Material aufkaschiert. Es besteht auch die Möglichkeit, die Elektrodeneinheit im Thermoverfahren auf die Kunststofffolie aufzukaschieren.

Wird die Elektrodeneinheit in einem Ganzkörper- oder Teilkörperanzug angeordnet, weist dieser Anzug vorteilhaft außen eine nicht leitfähige Schicht, beispielsweise eine Schicht aus Polyurethan auf. Die Elektrodeneinheit kann eine Verbindung zum Anzug beispielsweise mittels Verkleben oder auch mittels eines Thermoverfahrens aufweisen. Besonders vorteilhaft sind die Kunststofffolien aus einem dünnen flexiblen Material gebildet, welches eine hohe Leitfähigkeit aufweist, beispielsweise mit einem Widerstand von weniger als 100 Ohm pro 10 cm Länge der Elektrode.

Die elektrisch leitfähigen Fasern, die beispielsweise eingestickt, eingelegt oder eingedruckt werden, können beispielsweise aus Edelstahl oder Kupfer bestehen.

Das wenigstens eine Unterfütterungsteil, welches in der wenigstens einen Tasche angeordnet wird, ist vorteilhaft derart ausgebildet, dass es einen homogenen Anpressdruck oder einen punktuellen Anpressdruck der Elektrodeneinheit auf der Haut des Trägers erzeugt.

Wie schon ausgeführt, kann das wenigstens eine Unterfütterungsteil auch derart ausgebildet sein, dass es zur Entlastung des durch die Vorrichtung aufgebrachten Anpressdruckes der Elektrodeneinheit ausgebildet ist. Ist das wenigstens eine Unterfütterungsteil dafür vorgesehen, einen Anpressdruck auszuüben, kann der Anpressdruck punktuell oder flächig erzeugt werden.

Wie schon ausgeführt, können die Unterfütterungsteile unterschiedliche Härtegrade aufweisen. Die Unterfütterungsteile können auch starr ausgebildet sein.

Das textile Material der Vorrichtung, insbesondere des Kleidungsstückes ist vorteilhaft elastisch ausgebildet. Dies weist den Vorteil auf, dass der Träger sich mit der Vorrichtung, insbesondere mit dem Kleidungsstück sehr gut bewegen kann.

Grundsätzlich besteht auch die Möglichkeit, das textile Material der Vorrichtung, das heißt vorzugsweise des Kleidungsstückes nicht elastisch auszubilden. Dies kann beispielsweise besonders vorteilhaft vorgesehen, wenn die Vorrichtung als Armling oder Beinling oder Manschette ausgebildet ist.

Es besteht auch die Möglichkeit, das textile Material des Kleidungsstückes aus einer Kombination aus elastischem und nicht elastischem Material auszubilden.

Weitere Merkmale und Vorteile der Erfindung ergeben sich anhand der zugehörigen Zeichnungen, in der mehrere Ausführungsbeispiele der erfindungsgemäßen Vorrichtung dargestellt sind, ohne die Erfindung auf diese Ausführungsbeispiele zu beschränken. In den Zeichnungen zeigen:
- Fig. 1: einen Teilkörperanzug mit Elektrodeneinheiten;
- Fig. 2: eine Elektrodeneinheit im Querschnitt;
- Fig. 3: ein geändertes Beispiel einer Elektrodeneinheit im Querschnitt;
- Fig. 4: ein geändertes Beispiel einer Elektrodeneinheit im Querschnitt;
- Fig. 5: einen Querschnitt durch ein erstes Ausführungsbeispiel einer erfindungsgemäßen Vorrichtung;
- Fig. 6: eine Draufsicht auf die Vorrichtung gemäß Fig. 5;
- Fig. 7: einen Querschnitt durch ein zweites Ausführungsbeispiel der erfindungsgemäßen Vorrichtung;
- Fig. 8: eine Draufsicht auf das Ausführungsbeispiel der Fig. 7;
- Fig. 9: ein drittes Ausführungsbeispiel einer erfindungsgemäßen Vorrichtung im Querschnitt;
- Fig. 10: eine Draufsicht auf die erfindungsgemäße Vorrichtung gemäß Fig. 9;
- Fig. 11: einen Querschnitt durch ein weiteres Ausführungsbeispiel der erfindungsgemäßen Vorrichtung;
- Fig. 12: eine Draufsicht auf das Ausführungsbeispiel gemäß Fig. 11.

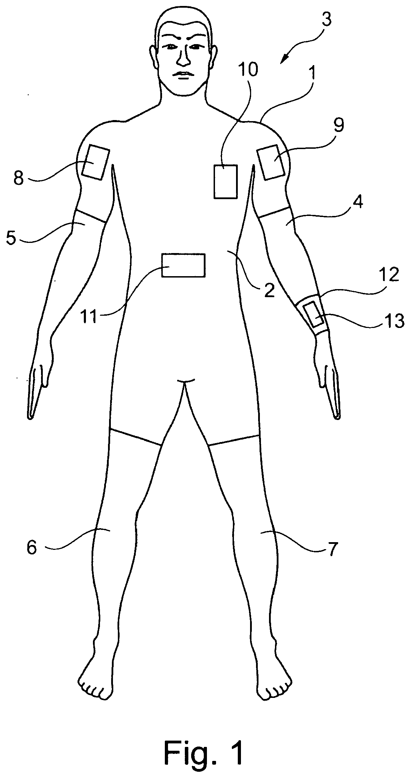

Fig. 1 zeigt einen Teilkörperanzug 1, der einen Rumpf 2 einer Person 3 abdeckt. Extremitäten 4 bis 7 bleiben teilweise frei von dem Teilkörperanzug 1. An dem Teilkörperanzug 1 sind Elektrodeneinheiten 8, 9, 10, 11 angeordnet. Die Elektrodeneinheiten 8, 9 sind im Oberarmbereich angeordnet, die Elektrode 11 im Bauchbereich und die Elektrode 10 im Herzbereich der Person 3. Darüber hinaus trägt die Person 3 an der Extremität 4, das heißt einem Arm, eine Manschette 12, an der eine Elektrodeneinheit 13 angeordnet ist. Die Elektrodeneinheiten 8, 9 und 11 können für eine Elektrostimulation verwendet werden. Die Elektrodeneinheiten 10, 13 können beispielsweise für eine Datenerfassung verwendet werden, beispielsweise für ein EKG.

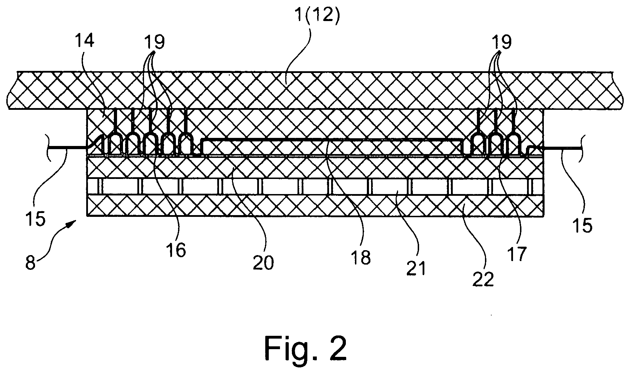

Fig. 2 zeigt die Elektrodeneinheit 8, die an dem Anzug 1 oder der Manschette 12 angeordnet ist. Die Elektrodeneinheit 1 besteht aus einer Grundschicht 14. In der Grundschicht 14 sind aus einem elektrisch leitfähigen Faden 15 zwei Elektroden 16, 17 eingestickt. Der Faden 15 tritt aus beiden Seiten der Grundschicht 14 hervor, damit ein elektrischer Stromkreis gebildet werden kann. Zwischen den Elektroden 16, 17 ist ebenfalls eine elektrisch leitfähige Verbindung 18 mit dem Faden 15 hergestellt. Der Faden 15 ist lediglich auf der dem Anzug abgewandten Seite der Grundschicht 14 eingestickt. Der Faden 15 wird mit Haltefäden 19, die nicht elektrisch leitfähig sind, in der Grundschicht 14 fixiert. Grundsätzlich besteht auch die Möglichkeit, dass die Elektroden 16, 17 aus zwei Fäden 15 gebildet werden, so dass zwei Stromkreise vorhanden sind. Die Elektroden 16, 17 sind in diesem Fall einzeln ansteuerbar.

Die Grundschicht 14 besteht aus Vlies und ist fest an dem Anzug 1 oder der Manschette 12 mittels Kleben, Thermopressverfahren oder dergleichen befestigt. Auf der dem Anzug 1 oder der Manschette 12 abgewandten Seite der Grundschicht ist eine Kunststoffschicht 20 angeordnet. An der Grundschicht 20 ist eine weitere Schicht aus einem Gewirk, im vorliegenden Fall aus einer Gaze 21 angeordnet. Darüber hinaus ist eine weitere Kunststoffschicht 22 vorgesehen.

Die Gaze 21 besteht vorteilhaft aus einem elastischen Material. Sie kann aus Polyester oder anderen Materialien bestehen.

Die Kunststofffolien 20, 22 sind vorteilhaft als Polyurethanfolie ausgebildet. In den Kunststoffschichten 20, 21 können entweder in der einen Schicht oder in der anderen Schicht oder in beiden Schichten elektrisch leitfähige Partikel wie zum Beispiel Graphit oder Silber (nicht dargestellt) angeordnet sein. Die Kunststoffschichten 20, 22 können auch aus thermoplastischem Material oder aus thermoplastischen Elastomeren wie auch aus leitfähigen Silikonen bestehen.

Die Schichten 14, 20, 21, 22 können in einem Heizpressverfahren miteinander fest verbunden werden. Sämtliche beschriebenen Schichten 1 (12, 14, 20, 21, 22) sind fest miteinander verbunden.

Die Fig. 2 zeigt den Aufbau der Schichten wie im Anspruch 1 mit dem Schichtaufbau b1) beschrieben.

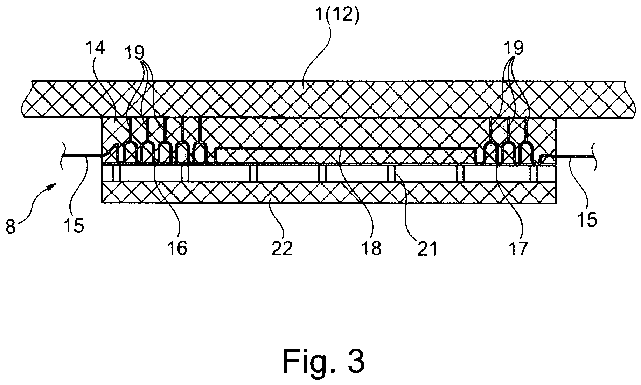

In Fig. 3 sind gleiche Teile wie in Fig. 2 mit den gleichen Bezugszeichen versehen. Die Elektrodeneinheit 8 besteht aus der Grundschicht 14 mit den eingestickten Elektroden 16, 17. Auf der Grundschicht 14 ist auf der dem Anzug 1 beziehungsweise der Manschette 12 abgewandten Seite die Gaze 21 angeordnet. Weiterhin ist eine Kunststoffschicht 22 vorgesehen. Dieser Aufbau entspricht dem Aufbau b2) des Anspruches 1. Die Materialien sind die Materialien, die zur Fig. 2 beschrieben wurden.

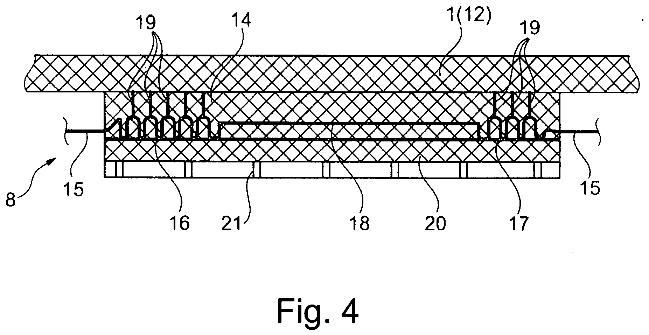

Fig. 4 zeigt einen weiteren möglichen Aufbau. Gleiche Teile wie in den Fig. 2 und 3 sind mit gleichen Bezugszeichen versehen. An dem Anzug 1 beziehungsweise der Manschette 12 ist die Grundschicht 14 mit den Elektroden 16, 17 angeordnet. Auf der dem Anzug 1 oder der Manschette 12 abgewandten Seite der Grundschicht 14 ist die Kunststoffschicht 20 angeordnet. Auf der Kunststoffschicht 20 ist die Gaze 21 angeordnet. Dieser Aufbau entspricht dem Aufbau b3) des Anspruches 1.

Die Grundschicht 14 kann aus einem Vlies, einem Gewirk, einem Gestrick oder dergleichen bestehen. Die Elektroden 16, 17 sind vorteilhaft eingestickt in die Grundschicht 14. Sie können jedoch auch gedruckt, eingelegt, festgeklebt oder dergleichen sein.

Durch die Anordnung des Schichtaufbaus auf der Grundschicht auf der Seite, die dem Anzug oder der Manschette abgewandt ist mit wenigstens einer Kunststoffschicht 20, 22 und der Gaze 21, wird eine flexible Elektrodeneinheit 8 gebildet, die auch durch langen Gebrauch mit wiederkehrenden Scher- und/oder Torsionskräften und/oder Streckungen und/oder Stauchungen lange haltbar ist.

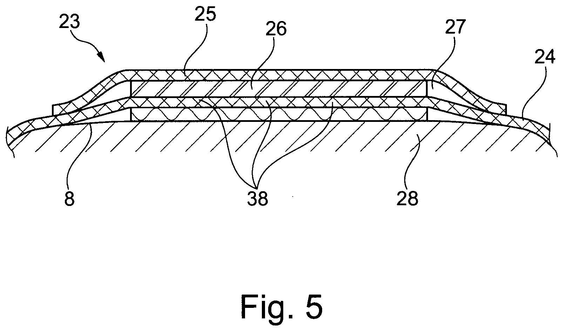

Fig. 5 zeigt einen Querschnitt durch eine erfindungsgemäße Vorrichtung 23, die im vorliegenden Fall beispielsweise als Anzug ausgebildet ist. Eine erste Lage 24 der Vorrichtung 23 trägt eine Elektrodeneinheit 8, die an einer Haut 28 eines Trägers (in Fig. 5 nicht dargestellt) anliegend ausgebildet ist. Die Vorrichtung 23 weist eine zweite Lage 25 auf, die mit der ersten Lage 24 eine Tasche 27 bildet. In der Tasche 27 ist ein Unterfütterungsteil 26 angeordnet. Das Unterfütterungsteil besteht beispielsweise aus thermoplastischem Polymer und kann als Spritzgussteil ausgebildet sein.

Durch die Anordnung des Unterfütterungsteiles 26 entsteht ein Bereich 38 mit einem erhöhten Anpressdruck. Die Unterfütterung 26 füllt die Tasche 27 fast vollständig aus und das Unterfütterungsteil 26 weist eine konstante Dicke auf und einen konstanten Härtegrad auf, so dass ein homogener Anpressdruck 38 entsteht. Die Elektrodeneinheit 8 wird damit mit einem homogenen Anpressdruck an die Haut 28 gedrückt.

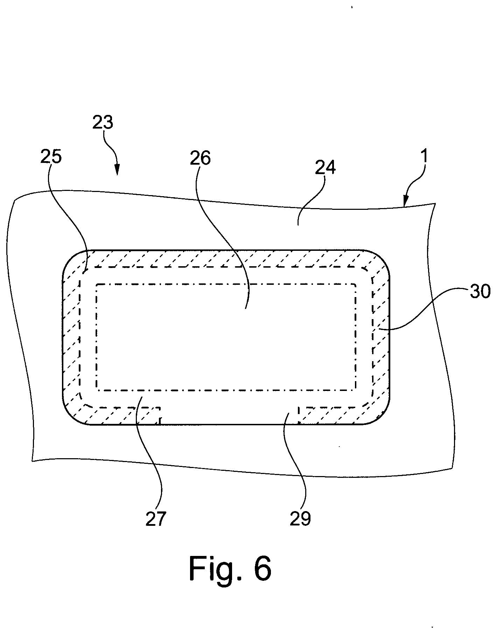

Fig. 6 zeigt die Vorrichtung 23, beispielsweise einen Teilbereich eines Anzuges 1. Die Vorrichtung 23 weist die erste Lage 24 auf. Eine zweite Lage 25 bildet mit der ersten Lage 24 eine Tasche 27. In einem Bereich 30 ist die erste Lage 24 mit der zweiten Lage 25 verklebt. Es wird hierdurch die Tasche 27 ausgebildet. Die Tasche 27 weist eine Öffnung 29 auf, durch die das Unterfütterungsteil 26 in der Tasche 27 angeordnet werden kann. Das Unterfütterungsteil ist strichliniert dargestellt.

Das Unterfütterungsteil 26 kann als relativ weiches Unterfütterungsteil 26 ausgebildet sein. Hierdurch wird die Homogenität des Anpressdruckes im Bereich 38 noch besser erreicht.

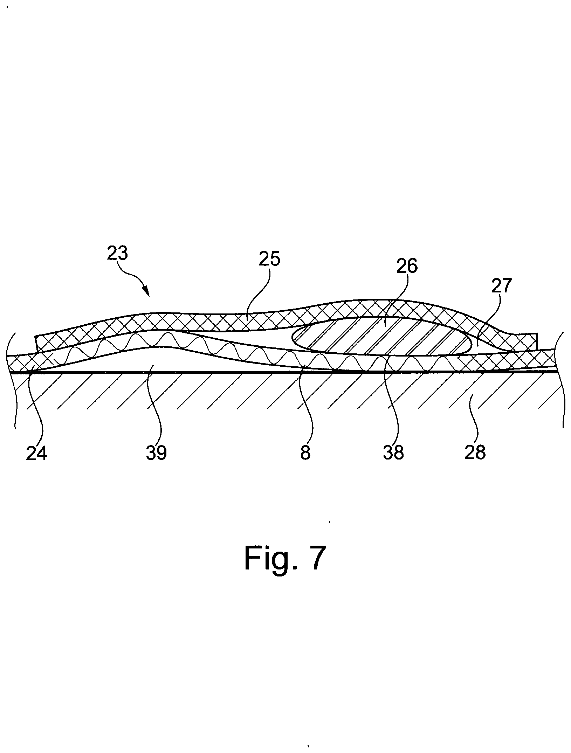

Fig. 7 zeigt ein weiteres Ausführungsbeispiel der Vorrichtung 23, beispielsweise eines Bodys. Die Vorrichtung 23 weist eine erste Lage 24 auf, in der gemäß Fig. 7 die Elektrodeneinheit 8 integriert angeordnet ist. Mit der zweiten Lage 25 bildet die erste Lage 24 eine Tasche 27, in der das Unterfütterungsteil 26 angeordnet ist. Das Unterfütterungsteil 26 füllt nicht die gesamte Tasche 27 aus. Aus diesem Grunde wird die Elektrodeneinheit 8 im Bereich 38 mit einem erhöhten Anpressdruck an die Haut 28 gedrückt. Im Bereich 39 liegt eine Entlastung des Anpressdruckes vor, so dass die Elektrodeneinheit 8 in diesem Bereich nicht oder lediglich mit einem geringen Anpressdruck an der Haut 28 anliegt.

Dieser Effekt kann noch erhöht werden, indem das Unterfütterungsteil 26 als ein hartes Unterfütterungsteil 26 ausgebildet ist. Hierdurch wird auch der Anpressdruck im Bereich 38 noch einmal deutlich erhöht.

Eine Entlastung des Anpressdruckes im Bereich 39 tritt dadurch auf, dass das Unterfütterungsteil 26 im Bereich 38 einen erhöhten Anpressdruck der Elektrodeneinheit erzeugt und damit im Bereich 39 der Anpressdruck verringert wird.

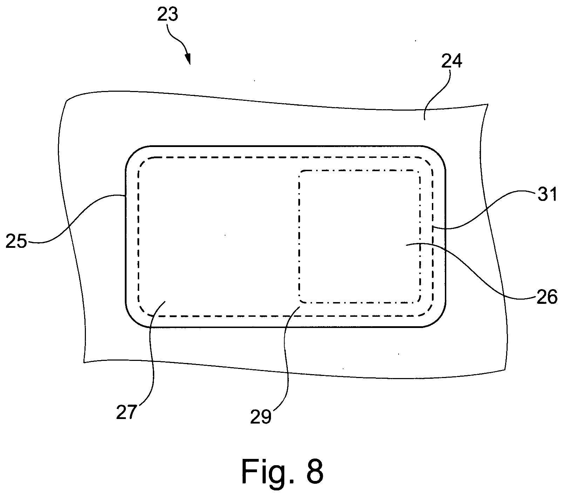

Gemäß Fig. 8 bildet die erste Lage 24 mit der zweiten Lage 25 eine Tasche 27. Die erste Lage 24 und die zweite Lage 25 sind miteinander vernäht. Es ist eine Naht 31 vorgesehen. Im Bereich der Öffnung 29 der Tasche 27 ist keine Naht 31 vorgesehen. Wie in Fig. 8 dargestellt, ist das Unterfütterungsteil 26 lediglich in einem Teilbereich der Tasche 27 angeordnet, wie es auch in der Fig. 7 dargestellt ist.

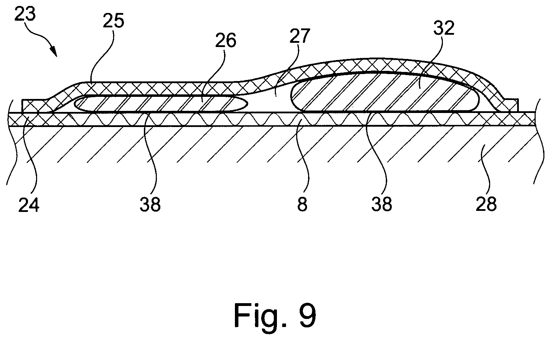

Fig. 9 zeigt die Vorrichtung 23, bei der eine erste Lage 24 mit einer zweiten Lage 25 eine Tasche 27 gebildet wird. Die Elektrodeneinheit 8 ist in der ersten Lage 24 integriert angeordnet, so dass die Elektrodeneinheit 8 an der Haut 28 anliegt.

In der Tasche 27 sind Unterfütterungsteile 26, 32 angeordnet, so dass zwei Bereiche 38 mit einem erhöhten Anpressdruck ausgebildet werden. Das Unterfütterungsteil 32 weist eine größere Dicke auf als das Unterfütterungsteil 26. Damit wird durch das Unterfütterungsteil 32 ein größerer Anpressdruck erzeugt als durch das Unterfütterungsteil 26.

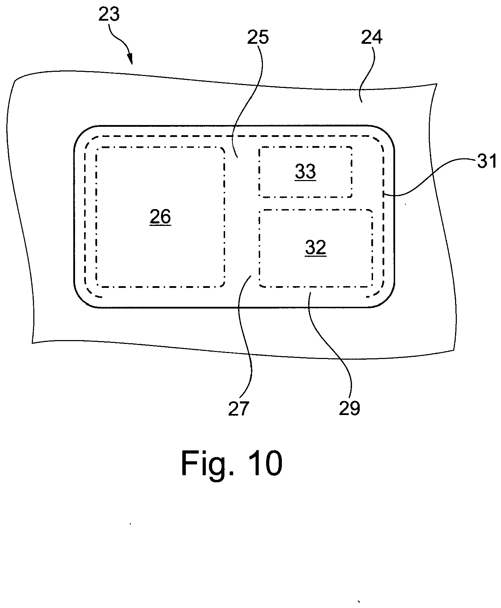

Fig. 10 zeigt die Vorrichtung 23 der Fig. 9.

Die erste Lage 24 ist mit der zweiten Lage 25 mittels einer Naht zu einer Tasche 27 zusammengefügt. Die Tasche 27 weist eine Öffnung 29 auf. In der Tasche 27 sind die Unterfütterungsteile 26, 32, 33 angeordnet. Wie in Fig. 9 dargestellt, weisen die Unterfütterungsteile 26, 32 unterschiedliche Dicken auf, so dass der Anpressdruck der Elektrodeneinheit 8 (in Fig. 9 dargestellt) unterschiedlich groß sein kann.

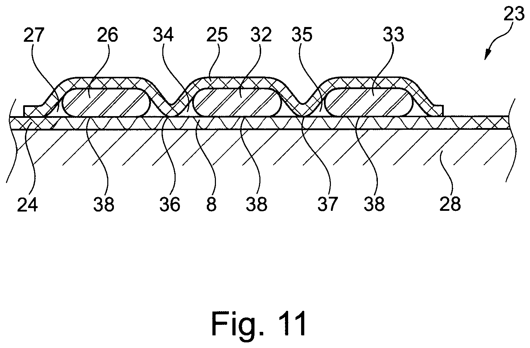

Fig. 11 zeigt die Vorrichtung 23 mit der ersten Lage 24, in der die Elektrodeneinheit 28 angeordnet ist. Die erste Lage 24 bildet mit der zweiten Lage 25 drei Taschen 27, 34, 35. In den Bereichen 36, 37 sind zusätzlich zu der Verbindung in Randbereichen zusätzliche Verbindungen zwischen der ersten Lage 24 und der zweiten Lage 25 vorgesehen. Die Verbindungen können durch Verkleben, Vernähen, Verschweißen oder dergleichen erzeugt werden.

In der Tasche 27 ist ein Unterfütterungsteil 26 angeordnet, in der Tasche 34 ist ein Unterfütterungsteil 32 angeordnet und in der Tasche 35 ist ein Unterfütterungsteil 33 angeordnet. In den Bereichen 38 wird durch die Unterfütterungsteile 26, 32, 33 ein erhöhter Anpressdruck der Elektrodeneinheit an der Haut 28 erzeugt.

Wie in Fig. 11 dargestellt, überdeckt die Tasche 27 den linken Bereich der Elektrodeneinheit, während die Tasche 35 den rechten Bereich der Elektrodeneinheit 8 nicht vollständig überdeckt.

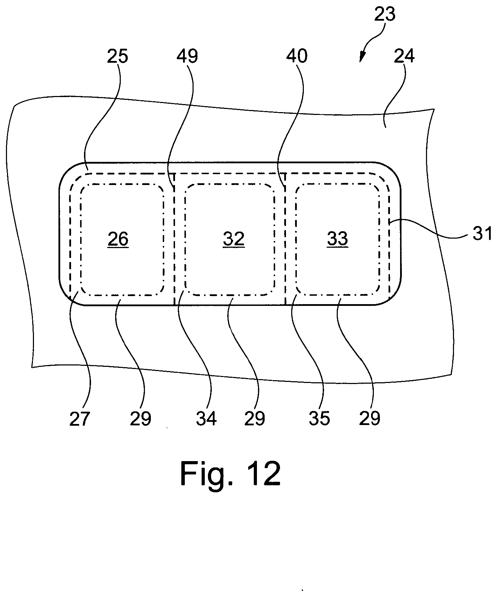

Fig. 12 zeigt die Vorrichtung 23 gemäß der Fig. 8 mit den drei Taschen 27, 34, 35. Die Taschen 27, 34, 35 sind durch zusätzliche Nähe 40 voneinander abgetrennt. Die Nähte 40 bilden eine Unterteilung der in den Fig. 5 bis 10 dargestellten Tasche 27.

Um die Taschen 27, 34, 35 auszubilden, ist die Naht 31 vorgesehen. Es verbleiben Öffnungen 29 der Taschen 27, 34, 35, um die Unterfütterungsteile 26, 32, 33 in den Taschen 27, 34, 35 anzuordnen und diese zu entnehmen.

Die dargestellten Ausführungsbeispiele der Fig. 5 bis 12 können beliebig miteinander kombiniert werden.

### Bezugszahlen

- 1: Teilkörperanzug
- 2: Rumpf
- 3: Person
- 4: Extremität (Arm)
- 5: Extremität (Arm)
- 6: Extremität (Bein)
- 7: Extremität (Bein)
- 8: Elektrodeneinheit
- 9: Elektrodeneinheit
- 10: Elektrodeneinheit
- 11: Elektrodeneinheit
- 12: Manschette
- 13: Elektrodeneinheit
- 14: Grundschicht
- 15: leitfähiger Faden
- 16: Elektrode
- 17: Elektrode
- 18: elektrisch leitfähige Verbindung
- 19: Haltefäden
- 20: Kunststoffschicht
- 21: Gaze
- 22: Kunststoffschicht
- 23: Vorrichtung
- 24: erste Lage
- 25: zweite Lage
- 26: Unterfütterungsteil
- 27: Tasche
- 28: Haut
- 29: Öffnung Tasche
- 30: verklebter Bereich
- 31: Naht
- 32: Unterfütterungsteil
- 33: Unterfütterungsteil
- 34: Tasche
- 35: Tasche
- 36: Verbindungsbereich
- 37: Verbindungsbereich
- 38: Bereich mit erhöhtem Anpressdruck
- 39: Bereich mit Entlastung des Anpressdruckes
- 40: Naht

## Patentansprüche

1. Vorrichtung mit wenigstens einer Elektrodeneinheit für eine Elektrostimulation oder eine Datenerfassung diagnostischer Geräte, wobei die Vorrichtung aus einem textilen Material gebildet ist, wobei die Vorrichtung aus wenigstens zwei Lagen gebildet ist, wobei in oder an einer ersten Lage die wenigstens eine Elektrodeneinheit angeordnet ist und eine zweite Lage derart an der ersten Lage angeordnet ist, dass die erste und die zweite Lage wenigstens eine Tasche bilden, wobei die Vorrichtung verschiedene austauschbare Unterfütterungsteile umfasst, die austauschbar in der wenigstens einen Tasche angeordnet werden können, und die jeweils unterschiedliche Dicken und/oder Härtegrade aufweisen, wobei in der wenigstens einen Tasche (27, 34, 35) wenigstens eines der Unterfütterungsteile (26, 32, 33) austauschbar angeordnet ist.

2. Vorrichtung nach Anspruch 1, **dadurch gekennzeichnet, dass** die wenigstens eine Tasche (27, 34, 35) derart an der Vorrichtung (23) angeordnet ist, dass die wenigstens eine Elektrodeneinheit (8) wenigstens teilweise von der wenigstens einen Tasche (27, 34, 35) überdeckt ausgebildet ist.

3. Vorrichtung nach einem der vorhergehenden Ansprüche, **dadurch gekennzeichnet, dass** das wenigstens eine Unterfütterungsteil (26, 32, 33) aus einem thermoplastischen Polymer, aus Schaumstoff, aus Vlies oder aus Silikon gebildet ist, oder dass das wenigstens eine Unterfütterungsteil (26, 32, 33) als Luftkissen oder Abstandsgewirk ausgebildet ist.

4. Vorrichtung nach einem der vorhergehenden Ansprüche, **dadurch gekennzeichnet, dass** die wenigstens eine Tasche (27, 34, 35) eine Fläche aufweist, die der Fläche der Elektrodeneinheit (8) entspricht.

5. Vorrichtung nach einem der vorhergehenden Ansprüche, **dadurch gekennzeichnet, dass** die wenigstens eine Tasche (27, 34, 35) eine größere oder eine kleinere Fläche aufweist als die Elektrodeneinheit (8).

6. Vorrichtung nach einem der vorhergehenden Ansprüche, **dadurch gekennzeichnet, dass** in der wenigstens einen Tasche (27, 34, 35) wenigstens ein Unterfütterungsteil (26, 32, 33) angeordnet ist, derart, dass die wenigstens eine Elektrodeneinheit (8) mit einem vorgegebenen Druck an der Haut (28) einer Person (3) anliegend ausgebildet ist.

7. Vorrichtung nach einem der vorhergehenden Ansprüche, **dadurch gekennzeichnet, dass** die zwei Lagen (24, 25) der Vorrichtung (23) zu wenigstens einer Tasche (27, 34, 35) zusammengefügt sind durch Kleben, Nähen und/oder Verschweißen.

8. Vorrichtung nach einem der vorhergehenden Ansprüche, **dadurch gekennzeichnet, dass** die wenigstens eine Tasche (27, 34, 35) wenigstens eine Unterteilung (40) aufweist.

9. Vorrichtung nach einem der vorhergehenden Ansprüche, **dadurch gekennzeichnet, dass** wenigstens eine zusätzliche Tasche vorgesehen ist, die außerhalb eines Bereiches mit einer Elektrodeneinheit (8) an der ersten Lage (24) angeordnet ist.

10. Vorrichtung nach einem der vorhergehenden Ansprüche, **dadurch gekennzeichnet, dass** die Vorrichtung (23) als Kleidungsstück, insbesondere als textiler Anzug oder als Teil eines textilen Anzuges ausgebildet ist.

11. Vorrichtung nach einem der vorhergehenden Ansprüche, **dadurch gekennzeichnet, dass** die Elektrodeneinheit (8, 9, 10, 11, 13) folgende Schichten aufweist:
a) Grundschicht (14) mit wenigstens einer in oder an der Grundschicht angeordneten Elektrode (16, 17), und
b) Schicht mit einem der folgenden Schichtaufbauten:
b1)
- Kunststoffschicht (20) und
- Schicht (21) aus Gewirk und
- Kunststoffschicht (22)
oder
b2)
- Schicht aus Gewirk (21) und
- Kunststoffschicht (22)
oder
b3)
- Kunststoffschicht (20) und
- Schicht (21) aus Gewirk
und dass die Schichten a) und b) (14, 20, 21, 22) fest miteinander verbunden sind, und dass die Grundschicht mit der ersten Lage identisch ist oder Teil der ersten Lage ist.

12. Vorrichtung nach Anspruch 11, **dadurch gekennzeichnet, dass** die Elektrode (16, 17) durch Sticken, Drucken, Nähen, Kleben und/oder Einlegen und Fixieren in oder an der Grundschicht (14) angeordnet ist.

13. Vorrichtung nach Anspruch 11 oder 12, **dadurch gekennzeichnet, dass** die Elektrode (16, 17) in oder an der vorgefertigten Grundschicht (14) angeordnet ist.

14. Vorrichtung nach einem der Ansprüche 11 bis 13, **dadurch gekennzeichnet, dass** die wenigstens eine Kunststoffschicht (20, 22) elektrisch leitfähig ausgebildet sind.

15. Vorrichtung nach einem der Ansprüche 11 bis 14, **dadurch gekennzeichnet, dass** die Schicht aus Gewirk aus Polyester gebildet ist.

16. Vorrichtung nach einem der Ansprüche 11 bis 15, **dadurch gekennzeichnet, dass** die wenigstens eine Schicht (21) aus Gewirk
- Gaze und/oder
- elastische Gaze und/oder
- gitterförmiges Material und/oder
- gitterförmiges elastisches Material
aufweist oder aus
- Gaze und/oder
- elastischer Gaze und/oder
- gitterförmigen Material und/oder
- gitterförmigen elastischem Material gebildet ist.

## Claims

1. Device comprising at least one electrode unit for electrostimulation or data acquisition from diagnostic equipment, the device being formed from a textile material, the device being formed from at least two plies, the at least one electrode unit being arranged in or on a first ply and a second ply being arranged on the first ply in such a way that the first and the second ply form at least one pocket, the device comprising various exchangeable lining parts which can be exchangeably arranged in the at least one pocket and which each have a different thickness or hardness, at least one of the lining parts (26, 32, 33) being exchangeably arranged in the at least one pocket (27, 34, 35).

2. Device according to Claim 1, **characterized in that** the at least one pocket (27, 34, 35) is arranged on the device (23) in such a way that the at least one electrode unit (8) is at least partly covered by the at least one pocket (27, 34, 35).

3. Device according to either of the preceding claims, **characterized in that** the at least one lining part (26, 32, 33) is formed from a thermoplastic polymer, from a foam, from a nonwoven or from a silicone or **in that** the at least one lining part (26, 32, 33) is in the form of an air cushion or a spacer fabric.

4. Device according to any of the preceding claims, **characterized in that** the at least one pocket (27, 34, 35) has a surface area corresponding to the surface area of the electrode unit (8).

5. Device according to any of the preceding claims, **characterized in that** the at least one pocket (27, 34, 35) has a larger or smaller surface area than the electrode unit (8).

6. Device according to any of the preceding claims, **characterized in that** at least one lining part (26, 32, 33) is arranged in the at least one pocket (27, 34, 35) such that the at least one electrode unit (8) rests against the skin (28) of a person (3) with a predefined pressure.

7. Device according to any of the preceding claims, **characterized in that** the two plies (24, 25) of the device (23) are joined together to form at least one pocket (27, 34, 35) by adhesive bonding, sewing and/or welding.

8. Device according to any of the preceding claims, **characterized in that** the at least one pocket (27, 34, 35) has at least one subdivision (40).

9. Device according to any of the preceding claims, **characterized in that** at least one additional pocket arranged on the first ply (24) beyond a region with an electrode unit (8) is provided.

10. Device according to any of the preceding claims, **characterized in that** the device (23) is in the form of a garment, in particular in the form of a textile suit or in the form of part of a textile suit.

11. Device according to any of the preceding claims, **characterized in that** the electrode unit (8, 9, 10, 11, 13) comprises the following layers:
a) base layer (14) having at least one electrode (16, 17) arranged in or on the base layer, and
b) layer having one of the following layer structures:
b1)
- plastics layer (20) and
- layer (21) of knitted fabric and
- plastics layer (22)
or
b2)
- layer of knitted fabric (21) and
- plastics layer (22)
or
b3)
- plastics layer (20) and
- layer (21) of knitted fabric
and **in that** layers a) and b) (14, 20, 21, 22) are permanently bonded to one another, and **in that** the base layer is identical to the first ply or is part of the first ply.

12. Device according to Claim 11, **characterized in that** the electrode (16, 17) is arranged in or on the base layer (14) by stitching, printing, sewing, adhesive bonding and/or inlaying and fixation.

13. Device according to Claim 11 or 12, **characterized in that** the electrode (16, 17) is arranged in or on the prefabricated base layer (14).

14. Device according to any of Claims 11 to 13, **characterized in that** the at least one plastics layer (20, 22) is electrically conductive.

15. Device according to any of Claims 11 to 14, **characterized in that** the layer of knitted fabric is formed from polyester.

16. Device according to any of Claims 11 to 15, **characterized in that** the at least one layer (21) of knitted fabric comprises
- gauze and/or
- elastic gauze and/or
- latticed material and/or
- latticed elastic material
or is formed from
- gauze and/or
- elastic gauze and/or
- latticed material and/or
- latticed elastic material.

## Revendications

1. Dispositif présentant au moins une unité d'électrode pour une électrostimulation ou une détection de données d'appareils diagnostiques, le dispositif étant formé à partir d'un matériau textile, le dispositif étant formé à partir d'au moins deux couches, ladite au moins une unité d'électrode étant agencée dans ou au niveau d'une première couche et une deuxième couche étant agencée sur la première couche de telle sorte que la première et la deuxième couche forment au moins une poche, le dispositif comprenant différentes parties de garniture remplaçables, qui peuvent être agencées de manière remplaçable dans ladite au moins une poche et qui présentent à chaque fois des épaisseurs différentes et/ou des degrés de dureté différents, au moins l'une des parties de garniture (26, 32, 33) étant agencée de manière remplaçable dans ladite au moins une poche (27, 34, 35).

2. Dispositif selon la revendication 1, **caractérisé en ce que** ladite au moins une poche (27, 34, 35) est agencée au niveau du dispositif (23) de telle sorte que ladite au moins une unité d'électrode (8) est réalisée de manière à être recouverte au moins partiellement par ladite au moins une poche (27, 34, 35).

3. Dispositif selon l'une des revendications précédentes, **caractérisé en ce que** ladite au moins une partie de garniture (26, 32, 33) est formée à partir d'un polymère thermoplastique, d'une mousse, d'un non-tissé ou d'une silicone ou **en ce que** ladite au moins une partie de garniture (26, 32, 33) est réalisée sous forme de coussin d'air ou de tricot d'écartement.

4. Dispositif selon l'une des revendications précédentes, **caractérisé en ce que** ladite au moins une poche (27, 34, 35) présente une surface qui correspond à la surface de l'unité d'électrode (8).

5. Dispositif selon l'une des revendications précédentes, **caractérisé en ce que** ladite au moins une poche (27, 34, 35) présente une surface supérieure ou inférieure à celle de l'unité d'électrode (8).

6. Dispositif selon l'une des revendications précédentes, **caractérisé en ce qu'**au moins une partie de garniture (26, 32, 33) est agencée dans ladite au moins une poche (27, 34, 35) de telle sorte que ladite au moins une unité d'électrode (8) est réalisée de façon à se placer avec une pression prédéfinie au niveau de la peau (28) d'une personne (3).

7. Dispositif selon l'une des revendications précédentes, **caractérisé en ce que** les deux couches (24, 25) du dispositif (23) sont assemblées en au moins une poche (27, 34, 35) par collage, couture et/ou soudure.

8. Dispositif selon l'une des revendications précédentes, **caractérisé en ce que** ladite au moins une poche (27, 34, 35) présente au moins une subdivision (40).

9. Dispositif selon l'une des revendications précédentes, **caractérisé en ce qu'**au moins une poche supplémentaire est prévue, qui est agencée à l'extérieur d'une zone présentant une unité d'électrode (8) au niveau de la première couche (24).

10. Dispositif selon l'une des revendications précédentes, **caractérisé en ce que** le dispositif (23) est réalisé comme une pièce de vêtement, en particulier comme une combinaison textile ou comme partie d'une combinaison textile.

11. Dispositif selon l'une des revendications précédentes, **caractérisé en ce que** l'unité d'électrode (8, 9, 10, 11, 13) présente les couches suivantes :
a) couche de base (14) présentant au moins une électrode (16, 17) agencée dans ou au niveau de la couche de base et
b) couche présentant l'une des structures de couche suivantes :
b1)
- couche en matériau synthétique (20) et
- couche (21) en tricot et
- couche en matériau synthétique (22)
ou
b2)
- couche en tricot (21) et
- couche en matériau synthétique (22)
ou
b3)
- couche en matériau synthétique (20) et
- couche (21) en tricot
et **en ce que** les couches a) et b) (14, 20, 21, 22) sont reliées solidement l'une à l'autre et **en ce que** la couche de base est identique à la première couche ou est une partie de la première couche.

12. Dispositif selon la revendication 11, **caractérisé en ce que** l'électrode (16, 17) est agencée dans ou au niveau de la couche de base (14) par broderie, impression, couture, collage et/ou insertion et fixation.

13. Dispositif selon la revendication 11 ou 12, **caractérisé en ce que** l'électrode (16, 17) est agencée dans ou au niveau de la couche de base (14) préfabriquée.

14. Dispositif selon l'une des revendications 11 à 13, **caractérisé en ce que** ladite au moins une couche en matériau synthétique (20, 22) est réalisée de manière électroconductrice.

15. Dispositif selon l'une des revendications 11 à 14, **caractérisé en ce que** la couche en tricot est formée à partir de polyester.

16. Dispositif selon l'une des revendications 11 à 15, **caractérisé en ce que** ladite au moins une couche (21) en tricot présente
- de la gaze et/ou
- de la gaze élastique et/ou
- un matériau en forme de treillis et/ou
- un matériau élastique en forme de treillis
ou est formée à partir
- de gaze et/ou
- de gaze élastique et/ou
- de matériau en forme de treillis et/ou
- de matériau élastique en forme de treillis.
